# EUROPEAN PATENT SPECIFICATION

(11) **EP 1 517 650 B1**
(45) Date of publication and mention of the grant of the patent: **29.11.2006**
(21) Application number: 03736518.6
(22) Date of filing: 01.05.2003
(51) Int. Cl.: A61F 2/06

(54) **STENT DELIVERY CATHETER WITH RETENTION BANDS**
KATHETER ZUM EINFÜHREN EINES STENTS MIT HALTEBÄNDERN
CATHETER DE POSE DE TUTEUR A BANDES DE RETENUE

(30) Priority: 20.06.2002 US 137909
(43) Date of publication of application: 30.03.2005
(73) Proprietor: Medtronic Vascular, Inc., Santa Rosa, CA 95403 (US)
(72) Inventor: FIFER, Dan, Windsor, CA 95492 (US)
(74) Representative: Hughes, Andrea Michelle
(86) International application number: PCT/US2003/013480
(87) International publication number: WO 2004/000168

(56) References cited:
- EP-A- 0 834 293
- FR-A- 2 753 907
- US-B1- 6 306 162

## Description

### FIELD OF THE INVENTION

The invention relates to intraluminal stenting, and in particular, to a stent delivery catheter having at least one elastic band being disposed around a balloon to reduce slippage between the stent and the balloon.

### BACKGROUND OF THE INVENTION

Intraluminal stenting is useful in treating tubular vessels in the body which are narrowed or blocked and it is an alternative to surgical procedures that intend to bypass such an occlusion. When used in endovascular applications, the procedure involves inserting a prosthesis into an artery and expanding it to prevent collapse of the vessel wall.

Percutaneous transluminal angioplasty (PTCA) is used to open coronary arteries which have been occluded by a build-up of cholesterol fats or atherosclerotic plaque. Typically, a guide catheter is inserted into a major artery in the groin and is passed to the heart, providing a conduit to the ostia of the coronary arteries from outside the body. A balloon catheter and guidewire are advanced through the guiding catheter and steered through the coronary vasculature to the site of therapy. The balloon at the distal end of the catheter is inflated, causing the site of the stenosis to widen. Dilation of the occlusion, however, can form flaps, fissures or dissections which may threaten re-closure of the dilated vessel. Implantation of a stent can provide support for such flaps and dissections and thereby prevent reclosure of the vessel. Reducing the possibility of restenosis after angioplasty reduces the likelihood that a secondary angioplasty procedure or a surgical bypass operation will be necessary.

A stent is typically a hollow, generally cylindrical device formed from wire(s) or a tube and the stent is commonly intended to act as a permanent prosthesis. A stent is deployed in a body lumen from a radially contracted configuration into a radially expanded configuration which allows it to contact and support a body lumen. The stent can be made to be either radially self-expanding or expandable by the use of an expansion device. The self expanding stent is made from a resilient material while the device-expandable stent is made from a material which is plastically deformable. A plastically deformable stent can be implanted during an angioplasty procedure by using a balloon catheter bearing the compressed stent which has been loaded onto the balloon. The stent radially expands as the balloon is inflated, forcing the stent into contact with the body lumen, thereby forming a support for the vessel wall. Deployment is effected after the stent has been introduced percutaneously, transported transluminally and positioned at a desired location by means of the balloon catheter.

A balloon of appropriate size is first used to open the lesion using a sufficient inflation pressure. The process can be repeated with a stent loaded onto a balloon. Direct stenting involves simultaneously performing angioplasty and stent implantation using a stent mounted on a dilatation balloon. After the balloon is withdrawn, the stent remains as a scaffold for the injured vessel.
In particular, the present invention relates to stents which can be delivered to a body lumen and which can be deployed at a treatment site by expanding the stent radially from a crimped state into an expanded state in which the stent supports the walls of the vessel at the treatment site. As noted above, the radial expansion is achieved by inflating a balloon on which the stent is located. One problem that can arise with this type of stent delivery system is that the stent may accidentally be displaced on the balloon as the delivery system negotiates torturous body vessels along its path to the treatment site. In order to ensure proper placement of the stent at the treatment site, one must avoid relative movement between the stent and the balloon. One means by which this risk of relative movement between the balloon and stent may be lessened is to form pillows on the balloon on either side of the stent to help prevent the stent from slipping off the balloon.

EP-A-0834293 discloses a stent delivery catheter having a balloon with integral bands around the balloon body.

### SUMMARY OF THE INVENTION

The present invention relates to a stent delivery catheter comprising: a flexible elongate shaft having proximal and distal ends and a lumen extending there through; a balloon having a generally cylindrical body with a length, the balloon being sealingly mounted about the shaft distal end in fluid communication with the lumen, the balloon being inflatable to plastically expand a stent to a nominal diameter for implantation of the stent in a vessel of a patient, the balloon being deflatable and wrappable about the shaft to form a deflated profile; and two or more elastic bands being disposed around the balloon body at spaced apart locations, and having i) a width substantially narrower than the length of the balloon body; ii) an ability to stretch elastically from a relaxed diameter smaller than the deflated profile to a stretched diameter larger than the nominal diameter, and iii) proximal and distal edges capable of mechanically engaging a stent from inside the stent; and characterized in that at least two of the elastic bands are joined to each other by one or more connector strips (50). The elastic bands provide improved grip between the balloon and a stent through increased friction and/or mechanical engagement with the edges of the band.

### BRIEF DESCRIPTION OF THE DRAWINGS

The present invention will be understood and appreciated more fully from the following detailed description (given by way of example only) taken in conjunction with the appended drawings in which:
FIG: I is a longitudinal view of a distal portion of a stent delivery catheter shown for background explanation, shown with the balloon partially inflated;
FIGS. 2-4 are enlarged views of modified forms of the catheter shown in FIG. 1;
FIG. 5 is a longitudinal view, partially in section, of a distal portion of a stent delivery catheter shown for explanation purposes, shown with a stent mounted thereon and the balloon partially inflated;
FIG. 6 is an arrangement of elastic bands mounted to a balloon catheter shown for explanation purposes;
FIG. 7 is an arrangement of elastic bands for application to a balloon catheter in accordance with the invention;
FIG. 8 is another alternative arrangement of elastic bands for application to a balloon catheter in accordance with the invention; and
FIG. 9 is yet another alternative arrangement of elastic bands for application to a balloon catheter in accordance with the invention.

### DETAILED DESCRIPTION OF THE INVENTION

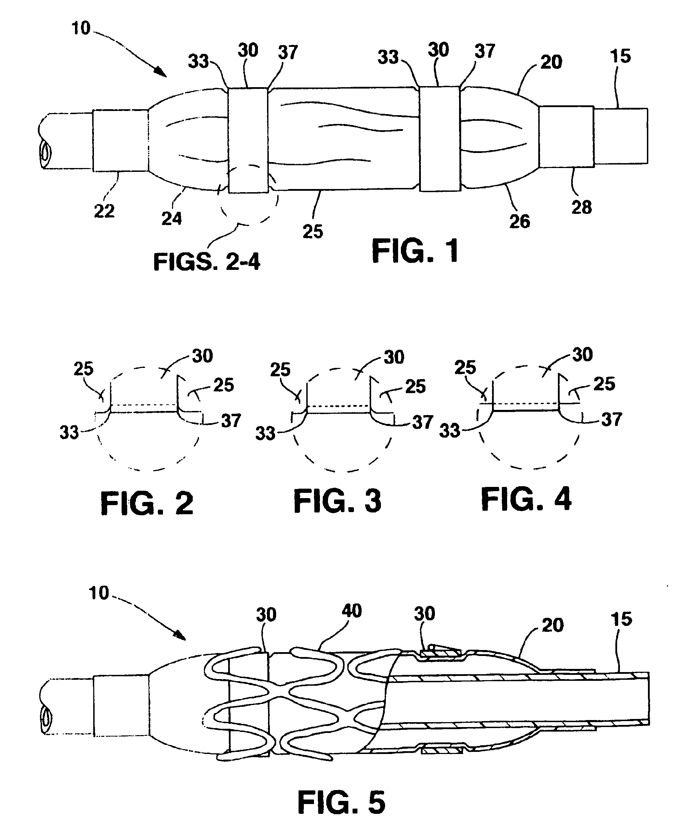

Applicant's invention is useful with any non self-expanding stent, such as those stents designed for delivery by a balloon. The stent may be generally cylindrical, and it may be mounted on a tubular balloon. FIG. 1 shows catheter 10, including shaft 15 and balloon 20, which can retain a stent thereon during delivery. One or more elastic bands 30 are mounted around generally cylindrical body 25 of balloon 20. Cylindrical body 25 may be centrally located between proximal and distal cones 24, 26, respectively. Proximal and distal cones 24, 26 terminate in proximal and distal ends 22, 28, respectively, which are adapted to be sealingly mounted on catheter shaft 15. Catheter shaft 15 comprises at least one lumen there through to provide fluid communication between balloon 20 and an inflation apparatus (not shown) connected to the proximal end of catheter 10. Catheter 10 may be fixed wire type, wherein balloon 20 is mounted adjacent the distal end of a shaft comparable to a steerable guide wire. Alternatively, catheter 10 may be of the rapid exchange type, having a short distal guidewire lumen, or catheter 10 may be of the over-the-wire type, having a full length guidewire lumen.

In FIG.1, balloon 20 is depicted in a partially inflated configuration as it would appear prior to full deflation and mounting a stent thereon, or after deployment of a stent. Balloon 20 can be made according to a variety of stretch blow molding processes that are well known to those skilled in the arts of dilatation and stent delivery balloons. Balloon 20 can be made from single or multiple layers of thermoplastics such as polyolefins, polyurethanes, polyamides, blends or copolymers that include these materials, or other polymers known to be suitable for dilatation and stent delivery balloons. The inelastic properties of such balloons cause wings or flaps to develop when the balloon is deflated. The balloon wings are wrapped compactly around shaft 15 to form as low a deflated profile as possible.

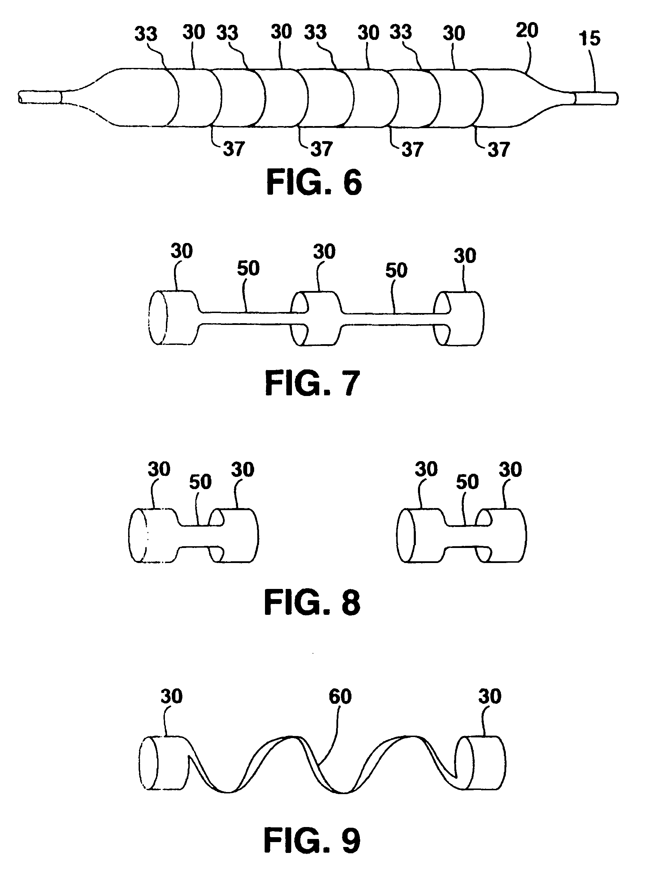

Two or more elastic bands 3 are located around balloon 20 to improve retention of stent 40 thereon, as depicted in FIG. 5. Bands 30 provide a high coefficient of friction relative to the adjacent material of balloon body 25, thus reducing slippage of stent 40 on balloon 20. Additionally, each band 30 has proximal and distal edges 33, 37, respectively, which can mechanically engage with the inside of stent 40, further enhancing retention of stent 40 on balloon 20. In the example shown in FIG. 6, balloon 20 is surrounded by a multiplicity of individual bands 30 disposed with spaces there between, such that numerous band edges 33, 37 are provided for gripping stent 40. If a stent dislodgement force is directed proximally, then distal band edges 37 can arrest proximal slippage of stent 40, especially by interlocking with proximally facing internal edges of a pattern formed in stent 40. Conversely, if a stent dislodgement force is directed distally, then proximal band edges 33 can arrest distal slippage of stent 40, especially by interlocking with distally facing internal edges of a stent pattern. To provide numerous gripping edges 33, 37 on catheter 10, the width of band 30 is substantially narrower than the length of body portion 25 such that body portion 25 can be encircled by a number of bands 30 with spaces there between.

In one example of the invention suitable for stent deployment within a coronary artery, balloon body portion 25 may measure 25 mm in length and be encircled by ten bands 30, each having a width of about 1.0 mm and spacing there between also measuring about 1.0 mm. The quantity and width of bands 30 selected for a particular stent delivery catheter according to the invention will be determined by the combination of frictional properties of the chosen band material and the number of band edges 33, 37 deemed necessary to achieve a desired stent retention force.

Elastic bands 30 have diameters, tensile strength, and elongation properties that have been selected such that bands 30 apply radial compressive force to balloon 20 at all achievable balloon diameters, which range from the deflated profile to the fully inflated, nominal stent deployment size. By having a relaxed diameter smaller than the deflated profile of catheter 10, elastic bands 30 can grip balloon 20 to retain stent 40 thereon, and to also keep individual bands 30 from slipping off of balloon 20 before, during or after stent deployment. Additionally, bands 30 will retain the balloon wings folded tightly around shaft 15.

Bands 30 may have tensile strength and elastic characteristics that provide minimal restriction of the inflation of balloon 20 to its nominal, stent deployment diameter. For bands 30 to maintain contact with stent 40 during expansion of stent 40 by balloon 20, bands 30 stretch radially, without significantly indenting balloon 20. For elastic band 30, the maximal stretched diameter that is achievable without plastic yield o r structural failure is larger than the nominal inflated diameter of balloon 20. For example, a stent delivery balloon 20 may have a nominal inflated diameter of 3.0 mm and a deflated profile of about 0.75 mm in diameter. During inflation of balloon 20, the length, or circumference, of elastic band 3 0 would stretch 400% without significantly constraining balloon expansion. The minimal restriction of balloon 20 during inflation may be achieved by making band 30 from an elastic material capable of elongation substantially greater than 400%.

FIGS. 2 - 4 illustrate a variety of conditions that may be achieved by selecting elastic bands of different sizes and/or different elongation properties. FIG. 2 shows band 30 not protruding above the outer surface of partially inflated balloon body 25, as may be accomplished by choosing a relatively tight-fitting elastic band 3 0. FIG. 2 c an also depict the fully inflated assembly that results from picking a n elastic band having a maximal stretched diameter only slightly larger than the nominal inflated diameter of balloon 20. Despite the flush surfaces of band 30 and balloon body 25, band edges 33, 37 are still exposed for mechanical engagement with stent 40 because balloon body 25 must constrict to fit within the inside diameter of band 30. Such an example may avoid slippage of stent 40 on balloon 20 by depending more on frictional engagement and less on mechanical engagement.

FIG. 3 shows band 30 protruding slightly above the outer surface of partially inflated balloon body 25, as may be accomplished by choosing an elastic band 30 that does not fit as tightly as the example of FIG. 2. FIG. 3 can also depict the fully inflated assembly that results from picking an elastic band having a maximal stretched diameter larger than the example illustrated in FIG. 2. In this example, band edges 33, 37 are further exposed for better mechanical engagement with stent 40, as compared to the previous example.

FIG. 4 shows band 30 protruding above the outer surface of partially inflated balloon body 25 by approximately the full wall thickness of band 30. This result may be accomplished by choosing an elastic band 30 that does not fit as tightly as the example of FIG. 3. FIG. 4 can also depict the fully inflated assembly that results from picking an elastic band having a maximal stretched diameter even larger than the example illustrated in FIG. 3. In this example, band edges 33, 37 are even further exposed for better mechanical engagement with stent40. Such an example may avoid slippage of stent 40 on balloon 20 by depending more on mechanical engagement and less on frictional engagement.

Bands 30 may be made of biocompatible elastic or elastomeric materials. Suitable elastic materials may include natural or synthetic rubbers, such as latex, silicone or other compounds. Suitable elastomeric materials may include thermoplastic elastomers (TPE's) such as Texin®, a polyether or polyester based polyurethane resin available from Bayer Corporation, Pittsburgh, Pennsylvania, USA or block copolymers, such as C-Flex®, a styrenic TPE comprising styrene ethylene/butylene styrene (SEBS) sold by Consolidated Polymer Technologies, Clearwater, Florida, USA. Bands 30 may be individually molded or cut from a molded or extruded tube. Bands 30 may also be cut from flat sheets of material, then rolled to form a circular band with its ends joined.

FIG. 7 illustrates a configuration of bands 30 according to the invention, wherein spaced-apart bands 30 are joined by longitudinal connectors 50. One or more connectors 50 join sequential bands 30, and may help maintain the desired position of bands 30 on balloon 20. Connectors 50 may be useful to prevent band 30 from slipping off of balloon 20. Longitudinal connectors 50 also provide additional surface area for frictional retention of stent 40. Longitudinal connectors 50 may be incorporated into a unitary molded configuration of bands, or connectors 50 may be made separately and subsequently joined to bands 30. In the latter example, connectors 50 need not be the same material as bands 30, especially since longitudinal connectors 50 do not have the same elongation requirements as bands 30.

FIG. 8 illustrates another alternative configuration of bands 30 according to the invention, wherein two separate pairs of spaced-apart bands 30 are joined by longitudinal connectors 50. FIG. 9 illustrates yet another alternative configuration of bands 30 according to the invention, wherein two spaced-apart b ands 30 a re joined by helical connector 60, which can encircle balloon body 25. Helical connector 60 provides the same function as longitudinal connectors 50. In addition, depending on the pitch angle of the helix, the edges of helical connector 60 may assist in stent retention in the same manner as band edges 33, 37, described *supra.* Because of its circumferential component, helical connector 60 needs some degree of radial stretchability, as is required of bands 30. Helical connector 60 may also be used with different combinations of elastic bands 30, similar to the alternative configurations shown in FIGS. 7 and 8.

Elastic bands 30 and connectors 50, 60 may be mounted over balloon body 25 by any of several methods. Bands 30 may be stretched and loaded onto a generally tubular mounting tool (not shown). The mounting tool may then be slipped over deflated, wrapped balloon 20, and bands 30 may be slid or rolled off of the tool into the desired compressive position on balloon 20. Alternatively, bands 30 made from TPE may be forced into the desired location around balloon 20 by use of heat shrink tubing in a method well known to those of skill in the arts of making medical catheters. In this example, TPE bands 30 are formed slightly oversized by molding or extrusion. After sliding bands 30 into position around balloon 20, heat shrink tubing is slipped over bands 30 and heat is applied to shrink the tubing and thermally reform bands 30 to a smaller diameter. Finally, the heat shrink tubing is removed.

Another alternative mounting process specific to silicone bands is to temporarily enlarge bands 30 for fitting over balloon 20. The temporary enlargement can be achieved by immersing silicone bands 30 in a silicone tube swelling fluid such as Lenium® TS, which is a proprietary blend of 3M™ HFE-7100 and octamethyltrisiloxane. Lenium TS is available from Petroferm Inc., Fernandina Beach, Florida, USA. After positioning enlarged bands 30 around balloon 20, the swelling fluid is permitted to evaporate, allowing bands 30 to shrink into engagement with balloon 20.

## Claims

1. A stent delivery catheter comprising:
a flexible elongate shaft (15) having proximal and distal ends and a lumen extending there through;
a balloon (20) having a generally cylindrical body with a length, the balloon being sealingly mounted about the shaft distal end in fluid communication with the lumen, the balloon being inflatable to plastically expand a stent to a nominal diameter for implantation of the stent in a vessel of a patient, the balloon being deflatable and wrappable about the shaft to form a deflated profile; and
two or more elastic bands (30) being disposed around the balloon body at spaced apart locations, and having
i) a width substantially narrower than the length of the balloon body;
ii) an ability to stretch elastically from a relaxed diameter smaller than the deflated profile to a stretched diameter larger than the nominal diameter, and
iii) proximal and distal edges capable of mechanically engaging a stent from inside the stent; and **characterized in that**
at least two of the elastic bands are joined to each other by one or more connector strips (50).

2. The stent delivery catheter of claim 1 wherein, when the balloon is wrapped about the shaft to form the deflated profile, the at least one band protrudes radially from the balloon body.

3. The stent delivery catheter of claim 1 or 2, wherein the ability of the at least one band to stretch elastically provides minimal restraint to the inflation of the balloon to the nominal diameter.

4. The stent delivery catheter of claim 3 wherein, when the balloon is inflated to the nominal diameter with an internal pressure sufficient to expand the stent, the at least one band protrudes radially from the balloon body

5. The stent delivery catheter of any preceding claim, wherein two of the elastic bands (30) are disposed, respectively, adjacent proximal and distal ends of the balloon body.

6. The stent delivery catheter of any preceding claim, wherein one or more of the connector strips (50) comprises an elastic material.

7. The stent delivery catheter of any preceding claim wherein the one or more connector strips extend longitudinally between the at least two elastic bands.

8. The stent delivery catheter of any of claims 1 to 6 wherein the one or more connector strips extend helically about the balloon body between the at least two elastic bands.

9. The stent delivery catheter of any preceding claim further comprising a plastically deformable tubular stent (40) disposed about the balloon body in mechanical engagement with at least one elastic band.

10. The stent delivery catheter of any preceding claim, wherein the at least two elastic bands have a coefficient of friction greater than the coefficient of friction of a balloon body portion adjacent the at least one elastic band.

## Patentansprüche

1. Katheter zum Einführen eines Stents, umfassend:
einen biegsamen, langen Schaft (15) mit einem proximalen und einem distalen Ende und einem sich dort hindurch erstreckenden Lumen;
einen Ballon (20) mit einem im wesentlichen zylindrischen Körper mit einer Länge, wobei der Ballon dichtend um das distale Schaftende herum in Fluidverbindung mit dem Lumen angeordnet ist, wobei der Ballon aufblasbar ist, um einen Stent plastisch auf einen Nenndurchmesser auszudehnen zur Implantierung des Stents in einem Gefäß eines Patienten, wobei der Ballon entleerbar und um den Schaft herumwickelbar ist, um ein entleertes Profil zu bilden; und
zwei oder mehr um den Ballonkörper herum an voneinander beabstandeten Stellen angeordnete Elastikbänder bzw. Gummibänder (30) mit
i) einer Breite, die im wesentlichen kleiner als die Länge des Ballonkörpers ist;
ii) einem elastischen Dehnvermögen von einem entspannten Durchmesser, der kleiner als das entleerte Profil ist, bis zu einem gedehnten Durchmesser, der größer als der Nenndurchmesser ist, und
iii) proximalen und distalen Kanten, die mit einem Stent vom Inneren dieses Stents aus mechanisch eingreifen können; und **dadurch gekennzeichnet, dass**
wenigstens zwei der Elastikbänder mittels eines oder mehrerer Verbindungsstreifen(s) (50) miteinander verbunden sind.

2. Katheter zum Einführen eines Stents gemäß Anspruch 1, wobei dann, wenn der Ballon um den Schaft gewickelt ist, um das entleerte Profil zu bilden, das wenigstens eine Band radial von dem Ballonkörper wegragt.

3. Katheter zum Einführen eines Stents gemäß Anspruch 1 oder 2, wobei die elastische Dehnfähigkeit des wenigstens einen Bandes das Aufblasen des Ballons auf den Nenndurchmesser nur minimal zurückhält.

4. Katheter zum Einführen eines Stents gemäß Anspruch 3, wobei dann, wenn der Ballon bei einem Innendruck, der ausreicht, um den Stent auszudehnen, auf den Nenndurchmesser aufgeblasen ist, das wenigstens eine Band radial von dem Ballonkörper wegragt.

5. Katheter zum Einführen eines Stents gemäß einem der vorangehenden Ansprüche, wobei zwei der Elastikbänder (30) benachbart zum entsprechenden proximalen bzw. distalen Ende des Ballonkörpers angeordnet sind.

6. Katheter zum Einführen eines Stents gemäß einem der vorangehenden Ansprüche, wobei ein oder mehrere Verbindungsstreifen (50) dehnbare Bestandteile bzw. Elastikbestandteile umfassen.

7. Katheter zum Einführen eines Stents gemäß einem der vorangehenden Ansprüche, wobei der bzw. die Verbindungsstreifen sich in Längsrichtung zwischen den wenigstens zwei Elastikbändern erstrecken.

8. Katheter zum Einführen eines Stents gemäß einem der Ansprüche 1 bis 6, wobei der bzw. die Verbindungsstreifen sich spiralförmig um den Ballonkörper herum zwischen den wenigstens zwei Elastikbändern erstrecken.

9. Katheter zum Einführen eines Stents gemäß einem der vorangehenden Ansprüche, umfassend einen plastisch deformierbaren, schlauchförmigen Stent (40), der in mechanischem Eingriff mit wenigstens einem Elastikband um den Ballonkörper herum angeordnet ist.

10. Katheter zum Einführen eines Stents gemäß einem der vorangehenden Ansprüche, wobei die wenigstens zwei Elastikbänder einen Reibungskoeffizienten aufweisen, der größer als der Reibungskoeffizient eines Abschnitts des Ballonkörpers ist, der zu dem wenigstens einen Elastikband benachbart ist.

## Revendications

1. Cathéter d'acheminement d'endoprothèse vasculaire, comportant :
un arbre allongé souple (15) ayant des extrémités proximale et distale et une lumière s'étendant à travers celui-ci ;
un ballon (20) ayant un corps généralement cylindrique avec une longueur, le ballon étant monté de manière étanche autour de l'extrémité distale de l'arbre en communication de fluide avec la lumière, le ballon pouvant être gonflé pour agrandir de manière plastique une endoprothèse vasculaire jusqu'à un diamètre nominal pour implantation de l'endoprothèse vasculaire dans un vaisseau d'un patient, le ballon pouvant être dégonflé et enroulé autour de l'arbre pour former un profil dégonflé ; et
deux bandes élastiques (30) ou plus étant disposées autour du corps du ballon au niveau d'emplacements espacés, et ayant
i) une largeur sensiblement plus étroite que la longueur du corps de ballon ;
ii) une aptitude à l'étirement de manière élastique à partir d'un diamètre relâché plus petit que le profil dégonflé vers un diamètre étiré plus grand que le diamètre nominal, et
iii) des bords proximal et distal capables de venir en prise mécaniquement avec une endoprothèse vasculaire à partir de l'intérieur de l'endoprothèse vasculaire, et **caractérisé en ce que**
au moins deux des bandes élastiques sont reliées l'une à l'autre par un ou plusieurs rubans de connexion (50).

2. Cathéter d'acheminement d'endoprothèse vasculaire selon la revendication 1, dans lequel le ballon est enroulé autour de l'arbre pour former le profil dégonflé, la au moins une bande faisant saillie radialement à partir du corps de ballon.

3. Cathéter d'acheminement d'endoprothèse vasculaire selon la revendication 1 ou 2, dans lequel l'aptitude de la au moins une bande à être étirée de manière élastique fournit un empêchement minimum au gonflage du ballon au diamètre nominal.

4. Cathéter d'acheminement d'endoprothèse vasculaire selon la revendication 3, dans lequel le ballon est dégonflé jusqu'au diamètre nominal avec une pression interne suffisante pour agrandir l'endoprothèse vasculaire, la au moins une bande faisant saillie radialement à partir du corps de ballon.

5. Cathéter d'acheminement d'endoprothèse vasculaire selon l'une quelconque des revendications précédentes, dans lequel deux des bandes élastiques (30) sont disposées, respectivement, adjacentes aux extrémités proximale et distale du corps de ballon.

6. Cathéter d'acheminement d'endoprothèse vasculaire selon l'une quelconque des revendications précédentes, dans lequel un ou plusieurs des rubans de connexion (50) sont constitués d'un matériau élastique.

7. Cathéter d'acheminement d'endoprothèse vasculaire selon l'une quelconque des revendications précédentes, dans lequel le ou les rubans de connexion s'étendent longitudinalement entre les au moins deux bandes élastiques.

8. Cathéter d'acheminement d'endoprothèse vasculaire selon l'une quelconque des revendications 1 à 6, dans lequel le ou les rubans de connexion s'étendent de manière hélicoïdale autour du corps de ballon entre les au moins deux bandes élastiques.

9. Cathéter d'acheminement d'endoprothèse vasculaire selon l'une quelconque des revendications précédentes, comportant en outre une endoprothèse vasculaire tubulaire pouvant être déformée de manière plastique (40) disposée autour du corps de ballon en prise mécanique avec au moins une bande élastique.

10. Cathéter d'acheminement d'endoprothèse vasculaire selon l'une quelconque des revendications précédentes, dans lequel les au moins deux bandes élastiques ont un coefficient de frottement supérieur au coefficient de frottement d'une partie de corps de ballon adjacente à la au moins une bande élastique.
